Europäisches Patentamt

European Patent Office

Office européen des brevets

(11) Publication number: **0 356 339 B1**

# EUROPEAN PATENT SPECIFICATION

(45) Date of publication of patent specification: **01.03.95**   (51) Int. Cl.⁶: **A61K 9/50**, A61K 39/145

(21) Application number: **89402343.1**

(22) Date of filing: **25.08.89**

Divisional application 94201844.1 filed on 25/08/89.

(54) **Influenza vaccine and novel adjuvants.**

(30) Priority: **25.08.88 US 236701**
**25.08.88 US 236702**
**23.08.89 US 397777**

(43) Date of publication of application:
**28.02.90 Bulletin  90/09**

(45) Publication of the grant of the patent:
**01.03.95 Bulletin  95/09**

(84) Designated Contracting States:
**AT BE CH DE ES FR GB GR IT LI LU NL SE**

(56) References cited:
**EP-A- 0 011 549**
**WO-A-85/04578**
**WO-A-86/05977**
**FR-A- 2 276 062**

**VACCINE, vol. 5, juin 1987, Bulterworth & Co. Ltd. (Publishers); G. GREGORIADIS et al., pp. 145-150/**

**THE LANCET, 08 novembre 1975; J.D. AL-MEIDA et al., pp. 899-901**

(73) Proprietor: **THE LIPOSOME COMPANY, INC. One Research Way**

**Princeton Forrestal Center
Princeton, NJ 08540 (US)**

(72) Inventor: **Popescu, Mircea C.**
**5 Parkway Avenue**
**Plainsboro, NJ 08536 (US)**
Inventor: **Recine, Marie S.**
**19 Hoffman Drive**
**Hamilton Twp., NJ 08690 (US)**
Inventor: **Alving, Carl L.**
**3 Newbold Court**
**Bethesda, MD 20817 (US)**
Inventor: **Estis, Leonard F.**
**56 Grafton Road**
**Upton, MA 01568 (US)**
Inventor: **Keyes, Lynn D.**
**56 Grafton Road**
**Upton, MA 01568 (US)**
Inventor: **Janoff, Andrew S.**
**1807 South Crescent Boulevard**
**Yardley, PA 19067 (US)**

(74) Representative: **Martin, Jean-Jacques et al**
**Cabinet REGIMBEAU**
**26, Avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 356 339 B1

## Description

Field of the Invention

This invention in the vaccine arts is concerned with an influenza immunizing dosage form comprising a liposome and an antigen of Influenza, particularly the hemagglutinin or bromelain fragment, wherein said liposome and antigen are present in an immunization dose. Additionally, a dosage form, including such form particularly adapted to producing an immune response, comprising dimyristoylphosphatidylcholine (DMPC)-/cholesterol liposomes, optionally in an aluminum hydroxide gel, and an antigen wherein said DMPC/cholesterol and antigen are present in an immunization dose, and method of use.

Background of the Invention

In the vaccine art antigens are introduced into an organism in a manner so as to stimulate an immune response in the host organism. The induction of an immune response depends on many factors among which are believed to include the chemical composition and configuration of the antigen, the immunogenic constitution of the challenged organism, and the manner and period of administration of the antigen. An immune response has many facets some of which are exhibited by the cells of the immune system, (e.g.,B-lymphocytes, T-lymphocytes, macrophages, and plasma cells). Immune system cells may participate in the immune response through interaction with antigen, interaction with other cells of the immune system, the release of cytokines and reactivity to those cytokines. Immune response is conveniently (but arbitrarily) divided into two main categories -- humoral and cell-mediated. The humoral component of the immune response includes production of immunoglobulins specific for the antigen. The cell-mediated component includes the generation of delayed-type hypersensitivity and cytotoxic effector cells against the antigen.

In some instances immune response is the result of an initial or priming dose of an antigen that is followed by one or more booster exposures to the antigen. Priming with relatively strong immunogens and liposomes is discussed in "Liposomal Enhancement of the Immunogenicity of Adenovirus Type 5 Hexon and Fiber Vaccines", Kramp, W.J. et al., Infection and Immunity, 25:771-773 (1979) and "Liposomes as Adjuvants with Immunopurified Tetanus Toxoid: the Immune Response", Davis, D. et al., Immunology Letters, 14:341-8 (1986/1987).

Ideally, an antigen will exhibit two properties, the capacity to stimulate the formation of the corresponding antibodies and the propensity to react specifically with these antibodies. Immunogens bear one or more epitopes which are the smallest part of an antigen recognizable by the combining site of an antibody or immunoglobulin.

In particular instances antigens or fractions of antigens or with particular presenting conditions the immune response precipitated by the desired antigen is inadequate or nonexistent and insufficient immunity is produced. This is particularly the case with peptide or other small molecules used as immunogens.

In such cases the vaccine art recognizes the use of substances called adjuvants to potentiate an immune response when used in conjunction with an antigen. Adjuvants are further used to elicit immune response sooner, or a greater response, or with less antigen or to increase production of certain antibody subclasses that afford immunological protection, or to enhance components of the immune response (e.g., humoral, cellular).

Well known adjuvants are Freund's Adjuvants (and other oil emulsions), Bortedella Pertussis, aluminum salts (and other metal salts), Mycobacterial products (including muramyl dipeptides), and liposomes. As used herein the term "adjuvant" will be understood to mean a substance or material administered together or in conjunction with an antigen which increases the immune response to that antigen. Adjuvants may be in a number of forms including emulsion (e.g., Freund's adjuvant) gels (aluminum hydroxide gel) and particles (liposomes) or as a solid material. Liposomal vaccines and adjuvancy are further discussed in U.S. Patent Application Ser. U.S. Serial Number 07/397,758 filed August 23, 1989, abandonned in favor of U.S. Serial Number 08/146,463, filed November 2, 1993

It is believed that adjuvant activity can be effected by a number of factors. Among such factors are (a) carrier effect, (b) depot formation, (c) altered lymphocyte recirculation, (d) stimulation of T-lymphocytes, (e) direct stimulation of B-lymphocytes and (f) stimulation of macrophages.

With many adjuvants adverse reactions are seen. In some instances adverse reactions include granuloma formation at the site of injection, severe inflammation at the site of injection, pyrogenicity, adjuvant induced arthritis or other autoimmune response, or oncogenic response. Such reactions have hampered the use of adjuvants such as Freund's adjuvant.

In particular embodiments adjuvants are comprised of liposomes. U.S. Patent No. 4,053,585 issued October 17, 1977 to Allison et al. states that liposomes of a particular charge are adjuvants. Davis, D, et al., "Liposomes as Adjuvants with Immunopurified Tetanus Toxoid: Influence of Liposomal Characteristics", Immunology, 61:229-234 (1987) and; Gregoriadis, G. et al., "Liposomes as Immunological Adjuvants: Antigen Incorporation Studies", Vaccine, 5:145-151 (1987) discloses the immune response of liposome-incorporated tetanus toxoid. The toxoid was incorporated into liposomes composed of equimolar phospholipid and cholesterol. In the Davis and in the Gregoriadis papers, the liposomal immunogenic response was only minimally distinguishable from the response of free antigen. To distinguish the liposomal from free antigen response it was necessary for the authors to dilute the tetanus toxoid to minimal response amounts. The present invention adopts conditions of DMPC/cholesterol liposomes that yield a therapeutically effective immunological response.

Other substances such as immunomodulators (e.g., cytokines such as the interleukins) may be combined in adjuvants/vaccines as well.

Humoral immune response may be measured by many well known methods. Single Radial Immunodifussion Assay (SRID), Enzyme Immunoassay (EIA) and Hemagglutination Inhibition Assay (HAI) are but a few of the commonly used assays of humoral immune response.

SRID utilizes a layer of a gel such as agarose containing the immunogen being tested. A well is cut in the gel and the serum being tested is placed in the well. Diffusion of the antibody out into the gel leads to the formation of a precipitation ring whose area is proportional to the concentration of the antibody in the serum being tested.

EIA, also known as ELISA (Enzyme Linked Immunoassay), is used to determine total antibodies in a sample. The antigen is adsorbed to the surface of a microtiter plate. The test serum is exposed to the plate followed by an enzyme linked immunoglublin, such as IgG. The enzyme activity adherent to the plate is quantified by any convenient means such as spectrophotometry and is proportional to the concentration of antibody directed against the antigen present in the test sample.

HAI utilizes the capability of an antigen such as viral proteins to agglutinate chicken red blood cells (or the like). The assay detects neutralizing antibodies, i.e. those antibodies able to inhibit hemagglutination. Dilutions of the test serum are incubated with a standard concentration of antigen, followed by the addition of the red blood cells. The presence of neutralizing antibodies will inhibit the agglutination of the red blood cells by the antigen.

Tests to measure allergic and cellular immune response include determination of delayed-type hypersensitivity or measuring the proliferative response of lymphocytes to target antigen.

Liposomes are completely closed lipid bilayer membranes containing an entrapped aqueous volume. Liposomes may be unilamellar vesicles (possessing a single bilayer membrane ) or multilamellar vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

The original liposome preparation of Bangham, et al. (J. Mol. Biol., 1965, 13:238-252) involves suspending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles described by Papahadjopoulos et al. (Biochim. Biophys. Acta., 1968, 135:624-638), and large unilamellar vesicles. Small unilamellar vesicles have a diameter of about 100nm or less.

Unilamellar vesicles may be produced using an extrusion apparatus by a method described in Cullis et al., PCT Application No. WO 87/00238, published January 16, 1986, entitled "Extrusion Technique for Producing Unilamellar Vesicles" incorporated herein by reference. Vesicles made by this technique, called LUVETS, are extruded under pressure once or a number of times through a membrane filter. LUVETs will be understood to be included in the term "unilamellar vesicle".

Another class of multilamellar liposomes are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4,588,578 to Fountain, et al. and frozen and thawed multilamellar vesicles (FATMLV) wherein the vesicles are exposed to at least one freeze and thaw cycle; this procedure is described in Bally et al., PCT Publication No. 87/00043, January 15, 1987, entitled "Multilamellar Liposomes Having Improved Trapping Efficiencies". U.S. Patent No. 4,721,612 to Janoff et al. describes steroidal liposomes for a variety of uses.

EP 0 356 339 B1

The teachings of these references as to preparation and use of liposomes are incorporated herein by reference.

Summary of the Invention

In one aspect this invention includes an influenza immunizing dosage form comprising a liposome and an antigen of Influenza wherein said liposome and antigen are present in an immunization dose. In a particular embodiment the antigen comprises the hemagglutinin fragment or the bromelain fragment. A particularly useful liposome comprises DMPC/cholesterol with particular reference to a mole ratio of from about 80 to about 20 DMPC to from about 20 to about 80 cholesterol, and more particularly wherein said ratio is from about 40:60 to about 60:40 and further wherein said liposome is a multilamellar vesicle such as one of substantially equal lamellar solute distribution (SPLV).

This invention includes a dosage form comprising an immunogen and a multilamellar liposome comprising DMPC/cholesterol in a immunization dose, in one embodiment further including aluminum adjuvants such as aluminum hydroxide gel. In one embodiment the liposome of the dosage form comprises a mole ratio of from about 80 to about 20 DMPC to from about 20 to about 80 cholesterol, and particularly wherein the ratio is from about 30:70 to about 70:30 and preferably 70:30. In specific embodiments the dosage form multilamellar liposome is of equal solute distribution (SPLV) and/or at least 1 micron in diameter and particularly a 70:30 mole ratio DMPC/cholesterol SPLV.

In particular embodiments of the dosage form the antigen is selected from the group comprising proteins, peptides, polysaccharides, bacterial fractions, viral fractions, protozoal fractions, synthetic peptides or lipopolysaccharides. Furthermore the dosage form may comprise an immunomodulator including a cytokine. Additionally the dosage form may comprise a suitable pharmaceutical carrier. Another aspect of this invention includes a composition for potentiating an immune response in an animal, including a human, comprising an immunization dose of a composition comprising an antigen and a multilamellar liposome comprising DMPC/cholesterol, and optionally further including aluminum adjuvants such as aluminum hydroxide gel. In one embodiment the liposomes comprise a mole ratio of from about 80 to about 20 DMPC to from about 20 to about 80 cholesterol, and particularly wherein the ratio is from about 30:70 to about 70:30. In specific embodiments of the method the multilamellar liposome is of equal solute distribution (e.g., SPLV) and/or at least about 1 micron in diameter and particularly a 70:30 mole ratio DMPC/cholesterol SPLV.

In particular embodiments the antigen is selected from the group comprising proteins, peptides, polysaccharides, bacterial fractions, viral fractions, protozoal fractions, synthetic peptides or lipopolysaccharides. Furthermore in employing the method the dose may comprise an immunomodulator including a cytokine. Additionally the dosage form may comprise a suitable pharmaceutical carrier.

In particular embodiments, the antigen is selected from the group comprising proteins, peptides, polysaccharides, bacterial fractions, viral fractions, protozoal fractions, synthetic peptides or lipopolysaccharides. Furthermore in employing the method the dose may comprise an immunomodulator including a cytokine. Additionally the dose form may comprise a suitable pharmaceutical carrier.

A further embodiment of the invention is a composition for priming an immune response in an animal, including a human, comprising a priming immunization dose of a composition comprising an adjuvant which is a multilamellar liposome comprising DMPC/cholesterol and an adjuvant-obligatory immunogen (and optionally aluminum adjuvants such as aluminum hydroxide gel) such that administration of a booster dose of adjuvant-obligatory immunogen absent adjuvant further potentiates immune response. The composition for priming, in specific instances includes the liposome being an SPLV multilamellar vesicle and/or the liposome being at least about 1 micron in diameter, and preferably about 70:30 DMPC/cholesterol. Specific immunogens of the dosage form and the methods are influenza fractions such as hemagglutinin, parainfluenza 3 (fusion and hemagglutinin-neuraminidase), malaria sporozoite fractions, hepatitis (A, B, and non-A/non-B) fraction, meningococcus fractions, HIV fractions (all strains), and melanoma fractions. A method of conferring immunity on an animal, including a human, comprises the step of administering to such animal a therapeutically effective immunization course at least one element of which is administering an immunization dose of a composition comprising an antigen and a multilamellar liposome comprising DMPC/cholesterol. In one aspect of the method the composition further comprises aluminum adjuvant such as aluminum hydroxide gel. In this method a particular liposome comprises a mole ratio of from about 80 to about 20 DMPC to from about 20 to about 80 cholesterol, preferably from about 70:30 to about 30:70 and most preferably about 70:30 particularly wherein the liposome is of equal solute distribution (e.g.,SPLV) and including the liposome being at least about 1 micron in diameter.

4

In the the composition for conferring immunity on an animal the antigen can be selected from the group comprising proteins, peptides, polysaccharides, bacterial fractions, viral fractions, protozoal fractions, synthetic peptides or lipopolysaccharides. The composition can further comprising an immunomodulator such as a cytokine. The composition can further comprise a suitable pharmaceutical carrier.

In an additional aspect the invention includes a composition for potentiating an immune response in an animal, including a human, comprising an immunization dose of a composition comprising an immunogen and a liposome comprising DMPC/cholesterol 70:30 +/-5 (mole). The composition can further comprise aluminum adjuvant such as aluminum hydroxide gel. The liposomes of the composition include multilamellar liposomes such as SPLVs and unilamellar liposomes, and preferably wherein the liposomes are at least about 1 micron in diameter. In embodiments of this composition including unilamellar liposomes the antigen is selected from the group comprising proteins, peptides, polysaccharides, bacterial fractions, viral fractions, protozoal fractions, synthetic peptides or lipopolysaccharides and further an immunomodulator such as cytokine and a suitable pharmaceutical carrier.

Further entailed in this invention is a composition for priming an immune response in an animal, including a human, comprising a priming immunization dose of a composition comprising an adjuvant which is a liposome comprising DMPC/cholesterol 70:30 +/-5 (mole) and an adjuvant-obligatory immunogen such that administration of a booster dose of adjuvant-obligatory immunogen absent adjuvant potentiates immune response. The composition can further comprise aluminum adjuvant such as aluminum hydroxide gel. The liposomes of the composition include multilamellar liposomes such as SPLVs and unilamellar liposomes, and preferably wherein the liposomes are at least about 1 micron in diameter.

A method of conferring immunity on an animal, including a human, comprises the step of administering to such animal a therapeutically effective immunization course at least one element of which is administering an immunization dose of a composition comprising an antigen and a liposome comprising DMPC/cholesterol 70:30 +/-5 (mole). The composition of the method can further comprise aluminum adjuvant such as aluminum hydroxide gel. The liposomes of the composition include multilamellar liposomes such as SPLVs and unilamellar liposomes, and preferably wherein the liposomes are at least about 1 micron in diameter. In embodiments of this composition including unilamellar liposomes the antigen is selected from the group comprising proteins, peptides, polysaccharides, bacterial fractions, viral fractions, protozoal fractions, synthetic peptides or lipopolysaccharides and further an immunomodulator such as cytokine and a suitable pharmaceutical carrier.

Detailed Description of the Invention

It has now been discovered that such adjuvants of this invention comprise an influenza immunizing dosage form comprising a liposome and an antigen of Influenza wherein said liposome and antigen are present in an immunization dose. Such antigens of Influenza are the hemagglutinin fragment or the bromelain fragment.

In addition it has now been discovered that DMPC/cholesterol liposomes (particularly multilamellar liposomes) are particularly useful pharmaceutical adjuvants (such adjuvants are advantageously used in aluminum hydroxide gels). Further preferred are DMPC/cholesterol liposomes, both multilamellar and unilamellar wherein the DMPC/cholesterol ratio is 70:30 +/-5 (mole).

The dosage forms or compositions comprising an influenza antigen and a salt form of an organic acid derivative of a sterol as an adjuvant, are described herein for illustrative purposes only and are not part of this invention.

The various terms used to define concepts in immunology are often loosely defined or otherwise misused. For clarity, in the discussion of this invention the following definitions will be used:

"Antigen" shall mean a substance or material that is recognized specifically by antibody and/or combines with an antibody.

"Adjuvant" shall mean a substance or material to potentiate an immune response when used in conjunction with an antigen. Adjuvants are further used to elicit immune response sooner, or a greater response, or with less antigen. Immunogen-obligatory adjuvant refers to an antigen which alone is not immunogenic but becomes immunogenic with adjuvant.

"Immunogen" shall mean a substance or material (including antigens) that is able to induce an immune response alone or in conjunction with an adjuvant. Both natural and synthetic substances may be immunogens. An immunogen will generally be a protein, peptide, polysaccharide, nucleoprotein, lipoprotein, synthetic polypeptide, or hapten linked to a protein, peptide, polysaccharide, nucleoprotein, lipoprotein or synthetic polypeptide or other bacterial, viral or protozoal fractions. It will be understood that "immunogen" includes substances which do not generate an immune response (or generate a only therapeutically ineffective immune response) unless associated with an adjuvant (e.g., small peptides) which will be referred to as "adjuvant-obligatory" immunogens.

"Immune response" shall mean a specific response of the immune system of an animal to antigen or immunogen. Immune response may include the production of antibodies.

"Immunization conditions" shall mean factors which affect an immune response including amount and kind of immunogen or adjuvant delivered to a subject animal including a human, method of delivery, number of inoculations, interval of inoculations, the type of subject animal and its condition.

"Vaccine" shall mean a pharmaceutical formulation able to induce immunity.

"Immunity" shall mean a state of resistance of a subject animal including a human to a infecting organism or substance. It will be understood that infecting organism or substance is defined broadly and includes parasites, toxic substances, cancers and cells as well as bacteria and viruses. A Therapeutically Effective Immunization Course will produce the immune response such as that exhibited by production of specific antibodies and/or reactivity of immune cells to antigen.

"Immunization dose" shall mean the amount of antigen or immunogen needed to precipitate an immune response. This amount will vary with the presence and effectiveness of various adjuvants.

This amount will vary with the animal and immunogen or antigen or adjuvant but will generally be between about 0.1ug/ml or less than about 500ug per inoculation. The immunization dose is easily determined by methods well known to those skilled in the art, such as by conducting statistically valid host animal immunization and challenge studies. See, for example, Manual of Clinical Immunology, H.R. Rose and H. Friedman, American Society for Microbiology, Washington, D.C. (1980). In some instances several immunization doses including booster doses will be administered to provide immunity, which collectively will be termed "Therapeutically Effective Immunization Course".

"Priming" shall mean the stimulation of a primary (as opposed to a secondary or later) response by an animal to an antigen. The primary response is characterized by the manufacture by the animal of antibody to the antigen, and ideally by the generation of a population of B-lymphocytes ad T-lymphocytes that respond to secondary or later immunogenic challenge -- even absent adjuvant --with a rapid and substantive production of antibodies. Based upon such response 1, 2, 3 or more booster doses of immunogen, absent adjuvant, will generate a therapeutically effective immune response to the antigen.

In particular embodiments of this invention the liposomes will have a net charge or be neutral. Charged and particularly negatively charged liposomes may display superior adjuvancy to neutral liposomes.

A preferred class of lipids for forming liposomes are those of cholesterol hemisuccinate ("CHS"), such as those with sodium ("CHS$_{sodium}$") or tris(hydroxymethyl) aminomethane ("CHS$_{tris}$") as the counter ion, which are generally negatively charged.

Salt forms of an organic acid derivative of a sterol may be used in the practice of the invention. Generally any sterol which can be modified by the attachment of an organic acid may be used in the practice of the present invention. For example, such sterols include but are not limited to cholesterol, vitamin D, phytosterols (including but not limited to sitosterol, campesterol, stigmasterol, and the like), steroid hormones, and the like.

Organic acids which can be used to derivatize the sterols include but are not limited to the carboxylic acids, dicarboxylic acids, polycarboxylic acids, hydroxy acids, amino acids and polyamino acids. Because the salt forms increase the water solubility of organic acids, any organic acid may be used to derivatize the sterols; however an advantage may be obtained if the organic acid moiety itself is water soluble. Such water soluble organic acid moieties include but are not limited to water-soluble aliphatic carboxylic acids such acetic, propionic, butyric, valeric acids and the like (N.B., up to four-carbon acids are miscible with water; the five-carbon free acid is partly soluble and the longer chain free acids are virtually insoluble); water-soluble aliphatic dicarboxylic acids such as malonic, succinic, glutaric, adipic, pimelic, maleic and the like (N.B., the shorter chains are appreciably more soluble in water; borderline solubility in water occurs at $C_6$ to $C_7$); and water-insoluble aromatic dicarboxylic acids such as hemimellitic, trimesic, succinimide, and the like; polycarboxylic acids; water-soluble hydroxy acids such as glycolic, lactic, mandelic, glyceric, malic, tartaric, citric, and the like (N.B., alpha-hydroxy acids containing a branched chain attached to the alpha-carbon of the carbonyl group would be less susceptible to hydrolysis and, therefore, advantageous in the practice of the present invention); and any of the amino acids and polyamino acids. The organic acid can be linked to an hydroxyl group of the sterol via an ester or an ether bond using conventional methods (see, for example, U.S. Pat. Nos. 3,859,047; 4,040,784; 4,042,330; 4,183,847; and 4,189,400). The salt forms of the derivatized sterols can be prepared by dissolving both the organic acid derivative of the sterol and the counterion of the salt (e.g., the free base of the salt) in an appropriate volatile solvent, and removing the solvent by evaporation or a similar technique leaving a residue which consists of the salt form of the organic acid derivative of the sterol. Counterions that may be used include, but are not limited to, tris, 2-amino-2-methyl-1,3-propanediol, 2-aminoethanol, bis-tris propane, triethanolamine, and the like to form the corresponding salt. In fact, the free base of an ionizable bioactive agent such as miconazole free base and the like may be used as the counterion.

CHS forms liposomes when added to an aqueous material. This can conveniently be performed at 20° - 25°C (room temperature) and atmospheric pressure. Agitation accelerates the process of liposome formation and is performed by such methods as vortexing, sonication or other methods well known in the art. If desired the resulting liposomes may be filtered or sized such as by passing through a filter stack such as a 0.4 or 0.2um filter (Nuclepore, Pleasanton, CA). Typically better adjuvant response is observed with greater amounts of lipid. Immunogens which partition into the liposome lamellae such as melanoma antigen in CHS liposomes may yield insufficient immunogenic responses without repeated inoculations and additional immuno stimulator. Without being bound by any particular theory it is believed that this partitioning results in the limitation of exposure of epitopes externally to the adjuvant liposomes. Immunogens may be modified by a number of methods well known in the art such as by amino acid addition or subtraction or conjugation with other moieties.

A preferred class of lipids for forming liposomes are those of dimyristoylphosphatidylcholine and cholesterol ("DMPC/cholesterol").

DMPC/cholesterol forms the required multilamellar liposomes over a wide range of proportions from about 100:1 (mole) to about 20:80. More preferred is about 70:30 to about 30:70, and yet further preferred is about 70:30. Additionally other lipids may be admixed with DMPC/cholesterol, such as dimyristoyl phosphatidylglycerol, dicetyl phosphate, phosphatidic acid, phosphatidylethanolamine, phosphatidylcholine and cholesterol hemisuccinate ("CHS"), such as those with sodium ("CHS$_{sodium}$") or tris(hydroxymethyl) aminomethane ("CHS$_{tris}$") as the counter ion.

Aluminum compounds are adjuvants well known in the art, and include aluminum hydroxide, aluminum phosphate, aluminum oxide or aluminum sulfate and will be termed collectively aluminum adjuvants. By way of example, aluminum hydroxide is widely used in diphtheria and tetanus toxoid vaccines as well as in veterinary applications. Aluminum hydroxide powder spontaneously forms a gel upon hydration. To prepare a vaccine containing aluminum hydroxide, commonly immunogen in aqueous buffer is added to the preformed gel. Such vaccines are referred to as being aluminum-adsorbed.

DMPC/cholesterol multilamellar liposomes of the SPLV process are preferred but any other type of liposome may be used. The SPLV process generally involves.rotoevaporation of lipids in solvent in a round bottom flask to form a thin film. The lipid film is then dispersed in a non-water miscible solvent such as ether or methylene chloride to which the aqueous solute (containing immunogen) is then added. The mixture is then sonicated while being dried by a stream of nitrogen gas which drives off the organic solvent. The resultant liposome paste is resuspended in aqueous buffer. U.S. Patent No. 4,522,803 to Lenk, et al. further describes this process and is incorporated herein by reference. If desired the resulting liposomes may be filtered or sized such as by passing through a filter stack such as a 0.4 or 0.2um filter (Nuclepore, Pleasanton, CA).

The resulting liposomes are conveniently administered in aqueous material. The volume of aqueous material will vary with the particular liposome to be administered and is not critical. Generally about 0.5ml is a convenient liposome dosage volume. Typically better adjuvant response is observed with greater amounts of lipid.

Suitable aqueous material for either sterol or DMPC/cholesterol liposomes is saline solution, phosphate buffer or other well known aqueous pharmaceutical diluents.

These liposomes are conveniently associated with an immunogenic amount of antigen or immunogen. This association is engendered by mixing, adsorption, encapsulation, co-formation or other methods well known in the art.

The adjuvant effect of the instant invention is seen from the results in Tables 1, 2 and 3 as to sterol liposomes and Tables 4, 5, and 6 as to DMPC/cholesterol liposomes and Table 7 as to both sterol and DMPC/cholesterol liposomes. Table 1A compares the antibody response in guinea pigs immunized with Influenza B/Ann Arbor hemagglutinin (HA) alone or formulations with CHS$_{tris}$ liposomes. HA elicits a particular level of antibody response without adjuvant, but this response is enhanced by adjuvant. CHS$_{tris}$ liposomes are seen from the results to potentiate this antibody production. In particular, neutralizing antibody responses as detected by HAI to 5 or 0.5 ug of HA are increased up to about 50-fold and 70-fold respectively when HA is administered with the steroidal adjuvant.

Table 1B shows the antibody response in guinea pigs immunized with the bromelain fragment of HA (HAB) in various formulations. HAB is generally non-immunogenic when administered alone and is exemplary of an adjuvant-obligatory immunogen. Use of steroidal adjuvant in the form of CHS increases the immune response on a par or better than complete Freund's adjuvant, especially the production of protective neutralizing antibodies as detected by HAI. This increase may be about 1400-fold. Furthermore, increasing the amount of adjuvant increases the immune response.

Table 2 demonstrates the importance of priming in generation of the immune response. HAB is seen to generate a weak immune response that is not greatly potentiated on secondary challenge, even with adjuvant of this invention. However, when HAB is administered in association with the adjuvant of this invention, here in the form of a CHS$_{tris}$ liposome a substantial immune response is generated upon application of a second inoculation of HAB, here in solution. It is an important aspect of this invention that priming of the subject animal with an immunogenic dose of adjuvant-obligatory immunogen and adjuvant permitted later booster doses to be highly effective wherein such booster doses did not contain adjuvant but were merely adjuvant-obligatory immunogen in solution. The priming of immune response with the adjuvant of this invention, permits booster administrations of adjuvant-obligatory immunogen without adjuvant, even when this immunogen would not have initially generated an immune response without coadministration with an adjuvant. In this embodiment of the invention it is important to note that any liposome can be used as a priming adjuvant, not just steroidal liposomes, for use with adjuvant-obligatory immunogens.

Table 3 discloses that superior results are obtained when the antigen is entrapped in an adjuvant liposome of the present invention and not merely mixed with the adjuvant liposome. This is particularly true when the priming response is considered. Table 3 in conjunction with Table 2 data indicates that entrapment is a potentiator of priming response, even more than a potentiator of secondary response. Table 3 shows also that changing the salt composition from tris to sodium does not alter the adjuvant effect provided by CHS liposomes.

Table 4 compares the antibody response in guinea pigs immunized with the bromelain fragment of Influenza B/Ann Arbor hemagglutinin (HAB) in various forms. HAB is an adjuvant-obligatory immunogen because it is poorly immunogenic when administered alone or when administered with aluminum hydroxide gel. The immunogenicity is greatly increased when HAB is entrapped within DMPC/cholesterol SPLV's at various mole ratios and lipid concentrations. Total anti-HA IgG responses detected by EIA are increased up to 5000 fold and protective neutralizing antibodies detected by HAI are increased up to 35 fold at 6 weeks when HAB is administered in DMPC/cholesterol SPLV's at 30:70 and 70:30 mole ratios, respectively. Increasing the lipid concentration also increases the adjuvant effect as demonstrated with 50:50 mole ratio formulations.

Table 5 demonstrates the adjuvant effect of DMPC/cholesterol SPLV's (70:30 mole ratio) on Influenza B/Ann Arbor hemagglutinin (HA). HA administered in free form is a relatively good immunogen at 5ug dose but elicits low antibody titers when administered at 0.5ug. Liposomal HA at both of these dosages generates responses which are up to 80 fold higher than free HA.

Table 6 demonstrates the adjuvant effect of DMPC/cholesterol SPLV's and aluminum hydroxide gel. As seen in Table 6 (Experiment 1), administration of HAB, with DMPC/cholesterol liposomes (200 mg starting lipid concentration) generates a strong anti-HA IgG response up to 100 fold greater and an HAI (neutralizing) antibody titer 10 fold greater than HAB alone. Increasing the starting lipid concentration to 500 mg increases the adjuvant effect even more (500 fold and 30 fold respectively). When the same formulations are administered with aluminum hydroxide gel, even these high titers are increased up to 3 times greater. The combined adjuvant effect for liposomes and aluminum hydroxide is clearly evident in (Table 6, Experiment II). As in the first experiment, liposomal HAB increases antibody responses up to 200 fold over free HAB (5ug dose). Administration with aluminum hydroxide gel substantially increases even these titers up to 5 times greater. Most striking is the observation that when a dose of 0.5ug liposomal HAB is administered with aluminum hydroxide a response equivalent to 5ug liposomal HAB is seen.

Table 7 shows the results obtained with HA used for vaccination after splitting the influenza virus with detergent. The results of Table 7 show that 5 mg HA in CHS$_{tris}$ liposomes induced a strong adjuvant effect regardless whether of this amount of antigen was entrapped in 13.6 or 1.9 mg of lipid. The same observation can be made in the case of DMPC/diol formulations.

Furthermore, 1:10 dilution of CHS$_{tris}$ formulation (0.5 ug HA) generated titers which were several fold higher than the non-entrapped antigen control at 0.5 or 5 ug HA.

Taken together the results showed that reduction of lipid content in formulation does not affect the adjuvant effect of liposomes.

Aluminum adjuvants are used in forms and proportions well known to those skilled in the art. Commercial preparations of aluminum hydroxide gel containing vaccines such as tetanus toxoids range from about 0.2 to about 1mg of aluminum/ml. The safe upper range is far higher for humans vaccines with as much as 15 mg or more of aluminum hydroxide per dose are known with no limit for veterinary applications.

Vaccines are conveniently administered in a dosage form. A "dosage form" will be understood to mean any pharmaceutically form of administering a vaccine including subcutaneous, oral, intramuscular, and ocular administration and utilizing vaccines in live, attenuated or synthetic or partial forms along with adjuvants and optionally immunomodulators such as cytokines. The combinations of the foregoing elements are prepared so that the dosage form is adapted to produce an immune response in the subject animal including a human as easily and effectively as possible.

The dosage forms including liposomal dosage forms resulting from the method of the present ivention can be used therapeutically in animals such as mammals, including man, in the treatment of infections or conditions which require the delivery of immunogen in its bioactive form. Such conditions include but are not limited to disease states such as those that can be treated with vaccines. Extracorporeal treatment of immunoresponsive tissues is also contemplated.

Dosage forms also include micelle forms of the adjuvant as well as adjuvant incorporated into gel such as aluminum gels, liquid crystals, powders, precipitates and solutions. In particular embodiments the dosage form can be a unit dosage form configured and adapted to a single administration.

The mode of administration of the dosage form may determine the sites and cells in the organism to which the dosage form will be delivered. The dosage forms including liposomal dosage forms of the present invention can be administered alone but will generally be administered in admixture with a pharmaceutical carrier selected with regard to the intended route of administration and standard pharmaceutical practice. The dosage forms may be injected parenterally, for example, intra-muscularly or subcutaneously. The dosage forms may also be administered via the oral route. For parenteral administration, they can be used, for example, in the form of a sterile aqueous solution which may contain other solutes, for example, enough salts or glucose to make the solution isotonic. Other uses, depending upon the particular properties of the preparation, may be envisioned by those skilled in the art.

For administration to humans in the preventive or curative treatment of disease states responding to vaccine therapy, the prescribing physician will ultimately determine the appropriate therapeutically effective dosage for a given human subject, and this can be expected to vary according to the age, weight, and response of the individual as well as the nature and severity of the patient's disease. The dosage of the antigen in liposomal dosage form will generally be about that or less than that employed for the free antigen. In some cases, however, it may be necessary to administer dosages outside these limits.

EXAMPLE 1

Preparation of Adjuvant Liposomes with Antigen

50mg of $CHS_{tris}$ (powdered) were placed into a 15ml test tube. 100ul of bromelain fragment of HA in aqueous buffer was added (673ug HAB, 0.01M phosphate buffered saline in 0.9%NaCl (wt.%)). The mixture was intermittently vortexed over a 2 hour period at 22.5°C +/-2.5°C and left until no large clumps were visible. The resultant liposomes were washed 3 times in 10 ml of aqueous buffer solution being separated each time by 15 minutes of centrifugation (10,000rpm, J-20 rotor (Beckman, Palo Alto, CA)). The final pellet was brought to 4.0ml in buffer and sealed in an amber vial under nitrogen.

EXAMPLE 2

Preparation of Adjuvant Liposomes with Antigen

500mg of $CHS_{tris}$ (powdered) were placed into a 15ml test tube. 1ml of HA in aqueous buffer was added (300ug HA, 0.01M phosphate buffered saline in 0.9%NaCl) The mixture was intermittently vortexed over a 2 hour period at 22.5°C +/-2.5°C. The resultant liposomes were washed 3 times in 10ml of aqueous buffer solution being separated each time by 15 minutes of centrifugation (10,000rpm, J-20 rotor (Beckman, Palo Alto, CA)). The final pellet was brought to 4.0ml in buffer and sealed in an amber vial under nitrogen.

EXAMPLE 3

Preparation of Adjuvant Liposomes with Antigen

500mg of $CHS_{tris}$ (powdered) were placed into a 15ml test tube. 1ml of bromelain fragment of HA in aqueous buffer was added (673ug HAB, 0.01M phosphate buffered saline in 0.9%NaCl) The mixture was intermittently vortexed over a 2 hour period at 22.5°C +/-2.5°C. The resultant liposomes were washed 3 times in 10ml of aqueous buffer solution being separated each time by 15 minutes of centrifugation (10,000rpm, J-20 rotor). The final pellet was brought to 4.0ml in buffer and sealed in an amber vial under nitrogen.

## EXAMPLE 4

Preparation of Adjuvant Liposomes with Antigen

500mg of $CHS_{tris}$ (powdered) were placed into a 15ml test tube. 2ml of bromelain fragment of HA in aqueous buffer was added (1,100ug HAB, 0.9%NaCl) The mixture was intermittently vortexed over a 2 hour period at 22.5°C+/-2.5°C. The resultant liposomes were washed 3 times in 10ml of 0.9%NaCl solution being separated each time by 15 minutes of centrifugation (10,000rpm, J-20 rotor). The final pellet was brought to 4.0ml in buffer and sealed in an amber vial under nitrogen.

## EXAMPLE 5

Preparation of Adjuvant Liposomes with Antigen

1500mg of $CHS_{tris}$ (powdered) were placed into a 15ml test tube. 3ml of bromelain fragment of HA in aqueous buffer was added (673ug HAB, 0.01M phosphate buffered saline in 0.9%NaCl) The mixture was intermittently vortexed over a 2 hour period at 22.5°C+/-2.5°C. The resultant liposomes were washed 3 times in 10ml of aqueous buffer solution being separated each time by 15 minutes of centrifugation (10,000rpm, J-20 rotor). The final pellet was brought to 4.0ml in buffer and sealed in an amber vial under nitrogen.

## EXAMPLE 6

Preparation of Adjuvant Liposomes with Antigen

500mg of $CHS_{sodium}$ (powdered) were placed into a 15ml test tube. 1ml of bromelain fragment of HA in aqueous buffer was added (673ug HAB, 0.01M phosphate buffered saline in 0.9%NaCl) The mixture was intermittently vortexed over a 2 hour period at 22.5°C+/-2.5°C. The resultant liposomes were washed 3 times in 10ml of aqueous buffer solution being separated each time by 15 minutes of centrifugation (10,000rpm, J-20 rotor). The final pellet was brought to 4.0ml in buffer and sealed in an amber vial under nitrogen.

## EXAMPLE 7

Adjuvancy Exhibited

Liposomes containing HAB were prepared as in Example 2. Entrapment values were determined by SRID and the liposomes were diluted in saline to a concentration of 10 ug protein/ml and were sealed in a glass vial with a rubber stopper and crimp seal.

Five 450-500g male Hartley guinea pigs (Buckberg Lab Animals, Landis Store, PA) were injected intramuscularly with 0.5 ml of the liposome suspension or with 0.5 ml of free HAB in the right hind leg (5 ug). At 4 weeks post immunization, the guinea pigs were lightly anesthetized with ether and approximately 4 ml of blood was drawn by cardiac puncture. The blood was allowed to clot at room temperature overnight. The blood was centrifuged, and the serum was drawn off and stored at 4°C until tested. The day after bleeding, the guinea pigs were again injected i.m. with 0.5 ml of free or liposomal HAB (5 ug), this time in the left hind leg. Blood was collected in the same manner to one year after the initial injection.

Total anti-HA IgG antibodies in the serum samples were determined by Enzyme Immunoassay (EIA) and neutralizing antibodies were determined by Hemagglutination Inhibition Assay (HAI). The results are shown in Tables 1, 2 and 3.

EXAMPLE 8

Preparation of Adjuvant Liposomes with Antigen

100mg of cholesterol and 400mg of DMPC were placed into a 500ml round bottom flask and suspended in 3ml chloroform and dried to a film by rotoevaporation. 20ml of anhydrous ether was added to the flask followed by 1.5ml of HA in aqueous buffer was added (915ug HA, 0.01M phosphate buffered saline in 0.9%NaCl) -- "aqueous buffer"). The mixture was covered loosely with foil and sonicated in a 40°C water bath while concurrently evaporating the ether with a gentle stream of nitrogen gas. The resultant lipid paste was thoroughly dried under nitrogen until no trace of ether was noted by smell. 10 ml of buffer was added to the flask and the liposome suspension was transferred to a 15ml test tube. The resultant liposomes were washed 3 times in 10ml of aqueous buffer being separated each time by 10 minutes of centrifugation (10,000rpm, J-20 rotor (Beckman, Palo Alto, CA)). The final pellet was brought to 6.0ml in buffer and sealed in an amber vial under nitrogen.

EXAMPLE 9

Preparation of Adjuvant Liposomes with Antigen

100mg of cholesterol and 400mg of DMPC were placed into a 500ml round bottom flask and suspended in 3ml chloroform and dried to a film by rotoevaporation. 20ml of anhydrous ether was added to the flask followed by 1.5ml of HAB in aqueous buffer was added (1,000ug HAB, 0.01M phosphate buffered saline in 0.9%NaCl). The mixture was covered loosely with foil and sonicated in a 40°C water bath while concurrently evaporating the ether with a gentle stream of nitrogen gas. The resultant lipid paste was thoroughly dried under nitrogen until no trace of ether was noted by smell. 10 ml of aqueous buffer was added to the flask and the liposome suspension was transferred to a 15ml test tube. The resultant liposomes were washed 3 times in 10ml of aqueous buffer solution being separated each time by 10 minutes of centrifugation (10,000rpm, J-20 rotor (Beckman, Palo Alto, CA)). The final pellet was brought to 6.0ml in buffer and sealed in an amber vial under nitrogen.

EXAMPLE 10

Preparation of Adjuvant Liposomes with Antigen

92mg of cholesterol and 108mg of DMPC were placed into a 100ml round bottom flask and suspended in 3ml chloroform and dried to a film by rotoevaporation. 10ml of anhydrous ether was added to the flask followed by 2.0ml of HAB in aqueous buffer was added (1,100ug HAB, 0.01M phosphate buffered saline in 0.9%NaCl). The mixture was covered loosely with foil and sonicated in a 40°C water bath while concurrently evaporating the ether with a gentle stream of nitrogen gas. The resultant lipid paste was thoroughly dried under nitrogen until no trace of ether was noted by smell. 10 ml of buffer was added to the flask and the liposome suspension was transferred to a 15ml test tube. The resultant liposomes were washed 3 times in 10ml of aqueous buffer solution being separated each time by 10 minutes of centrifugation (10,000rpm, J-20 rotor (Beckman, Palo Alto, CA)). The final pellet was brought to 6.0ml in buffer and sealed in an amber vial under nitrogen.

EXAMPLE 11

Preparation of Adjuvant Liposomes with Antigen

40mg of cholesterol and 160mg of DMPC were placed into a 100ml round bottom flask and suspended in 3ml chloroform and dried to a film by rotoevaporation. 10ml of anhydrous ether was added to the flask followed by 2.0ml of HAB in aqueous buffer was added (1,100ug HAB, 0.01M phosphate buffered saline in 0.9%NaCl). The mixture was covered loosely with foil and sonicated in a 40°C water bath while concurrently evaporating the ether with a gentle stream of nitrogen gas. The resultant lipid paste was thoroughly dried under nitrogen until no trace of ether was noted by smell. 10 ml of buffer was added to the flask and the liposome suspension was transferred to a 15ml test tube. The resultant liposomes were washed 3 times in 10ml of aqueous buffer solution being separated each time by 10 minutes of centrifugation (10,000rpm, J-20 rotor (Beckman, Palo Alto, CA)). The final pellet was brought to 6.0ml in buffer and sealed in an amber vial under nitrogen.

Example 12

Preparation of Gel Admixed with Liposomes

Aluminum hydroxide gel, 2% (Alhydrogel™; Connaught Laboratories, Inc., Swiftwater, PA) containing 7.29mg/ml aluminum was used in conjunction with 1.02ml of the liposomes of this invention prepared as in Example 10. The liposomes were admixed with 0.67ml aluminum hydroxide gel and 5.31ml of saline. The final aluminum concentration was 0.7mg/ml and the HAB concentration was 10ug/ml. The mixture was sealed in a glass vial with rubber stopper and crimp seal.

EXAMPLE 13

Adjuvancy

Liposomes containing HAB were prepared as in Example 9. Entrapment values were determined by SRID and the liposomes were diluted in saline to a concentration of 10 ug protein/ml and were sealed in a glass vial with a rubber stopper and crimp seal.

Five 450-500g male Hartley guinea pigs (Buckberg Lab Animals, Landis Store, PA) were injected intramuscularly with 0.5 ml of the liposome suspension or with 0.5 ml of the liposome suspension or with 0.5 ml of free HAB in the right hind leg (5ug). At 4 weeks post immunization, the guinea pigs were lightly anesthetized with ether and approximately 4 ml of blood was drawn by cardiac puncture. The blood was allowed to clot at room temperature overnight. The blood was centrifuged, and the serum was drawn off and stored at $4°C$ until tested. The day after bleeding, the guinea pigs were again injected i.m. with 0.5 ml of free or liposomal HAB (5ug), this time in the left hind leg. Blood was collected in the same manner up to one year after the initial injection.

Total anti-HA IgG antibodies in the serum samples were determined by Enzyme Immunoassay (EIA) and neutralizing antibodies were determined by Hemagglutination Inhibition Assay (HAI). The results are shown in Tables 4, 5 and 6.

Example 14

Melanoma Procedures

Liposomes containing ganglioside $GD_3$ (kindly provided by Dr. P Livingston, Sloan Kettering Institute for Cancer Research, New York, New York) were prepared at 10:1 and 100:1 (w/w) lipid to antigen ($GD_3$) ratio using 10mg lipid to 1 mg $GD_3$ and 150mg lipid to 1.5 mg $GD_3$. Liposomes were prepared using either $CHS_{tris}$ or DMPC/cholesterol (70:30 mole percent).

$GD_3$ antigen suspended in phosphate buffered saline (PBS but without $Ca^{++}$ or $Mg^{++}$) at pH 7.2 was entrapped in either $CHS_{tris}$ lipid liposomes or DMPC/cholesterol (70:30 mole percent) lipid liposomes. $CHS_{tris}/GD_3$ MLV liposomes were made by Method A (below) and DMPC/cholesterol/$GD_3$ SPLV liposomes by Method B (below).

Mice were injected 4 times with the liposomal antigen preparation and antibody level tested. Antibody to melanoma $GD_3$ was produced. It was noted however that other melanoma vaccine preparations yielded higher antibody titers in mice than the instant preparation by the regimen described herein.

Method A

10 mg $CHS_{tris}$ (powdered) and 1 mg $GD_3$ (10:1 ratio) (wt/wt) in 50 ul PBS were placed in a test tube and hydration of lipids allowed for 2 hours at room temperature with intermittent vortexing. The lipidic material was resuspended in 10 ml PBS with $Ca^{++}$ or $Mg^{++}$ and centrifuged at 13,000 rpm for 30 minutes, two times. The final liposome pellet was resuspended in 2.5 ml PBS. The liposome preparation was sealed in an amber vial under nitrogen until testing. An additional formulation at 100:1 lipid/antigen ratio was prepared as above, using 150 mg $CHS_{tris}$ and 1.5 mg $GD_3$ in 300 ul PBS.

12

Method B

2 mg cholesterol and 8 mg DMPC in 0.5 ml chloroform were dried by rotoevaporation in a 25 ml roundbottom flask, resolubilized in 2 ml anhydrous ether and mixed with 1.5 mg $GD_3$ in 60 ul PBS. The resulting mixture was sonicated at 40°C under a stream of $N_2$ to form a lipid paste which was further dried under $N_2$. The dry material was resuspended in 10 ml PBS and centrifuged at 10,000 rpm for 10 minutes, three times. The final liposome pellet was brought to 2.28 ml and supplemented with Alhydrogel (0.7 mg Al/ml). An additional formulation at 100:1 lipid/antigen ratio was prepared as above using 120 mg DMPC, 30 mg cholesterol, and 1.5 mg $GD_3$ in 0.45 ml PBS.

Example 15

Additional Immune Response Studies

To determine further the role of the dose of both HA (split antigen) and lipid, guinea-pigs were inoculated with liposome formulations at 5 or 0.5 ug HA each entrapped in various amount of lipid (Table 7).

Two $CHS_{tris}$ formulations were prepared as described in Example 2 to give a dose of 5.0 by HA and either 13.6 mg lipid (MLV, $CHS_{tris}$, H) or 1.9 mg lipid as multilamellae vesicles of CHS (MLV, $CHS_{tris}$, L). The "H" and "L" designations refer to high and low amount of lipid in particular dosages. The demarcation of high and low is arbitrary but convenient in conceptualizing any role that the relative amount of lipid plays in vaccine efficacy.) Two DMPC/CHOL formulations were prepared as described in Example 8 to give a dose of 5.0 mg HA and either 9.7 mg lipid of stable plurilamellar vesicles, dimyristoylphosphatidyl-choline/cholesterol (SPLV, DMPC/C, H) or 2.9 mg lipid (SPLV, DMPC/C, L).

In addition each H formulation exhibiting a relatively high amount of lipid per 0.5 ml inoculum was further diluted 1:10 in PBS and the resulting suspensions containing 0.5 ug HA and either 1.4 mg $CHS_{tris}$ or 1.0 mg DMPC/CHOL also inoculated in the same vial. Inoculation of guinea pigs and determination of antibody by EIA or HAI were performed as described in Example 7 and Example 15.

Table 1

Immunogenicity Enhancement of HA and HAB via Steroidal Adjuvant

ANTI-HA ANTIBODY TITERS (Units/ml)

| Formulation | Example No. | Dose (ug) | Starting Lipid Concentration (mg) | Primary 4 week EIA | HAI | Secondary 6 week EIA | HAI | 8 week EIA | HAI | 12 week EIA | HAI | 25 week EIA | HAI | 52 week EIA | HAI |
|---|---|---|---|---|---|---|---|---|---|---|---|---|---|---|---|
| **A.** | | | | | | | | | | | | | | | |
| HA | | 5.0 | NA | 24.9 | 28.6 | 527.3 | 1810.2 | 306.4 | 452.6 | 141.3 | 226.3 | 92.4 | 148.9 | – | 100.8 |
| HA in CHS-TRIS MLV's | 2 | 5.0 | 500 | 853.9 | 1338.1 | 4047.9 | 9667.5 | 2520.8 | 1140.9 | 1039.2 | 1280.0 | 610.1 | 1280.0 | – | 403.2 |
| HA | | 0.5 | NA | 0.9 | 5.0 | 103.4 | 190.3 | 43.5 | 31.3 | 29.6 | 40.0 | 29.5 | 40.0 | – | 23.7 |
| HA in CHS-TRIS MLV's | 2 | 0.5 | 500 | 52.2 | 52.8 | 2643.8 | 8914.4 | 1669.0 | 2228.6 | 861.9 | 1114.3 | 624.1 | 970.1 | – | 640.0 |
| **B.** | | | | | | | | | | | | | | | |
| HAB | | 5.0 | NA | 0.4 | 5.0 | 1.8 | 5.0 | 1.4 | 5.0 | $<$1.5 | 9.1 | 1.4 | 6.3 | – | 5.0 |
| HAB in Complete Freund's Adjuvant | | 5.0 | NA | 1225.6 | 105.6 | 3775.6 | 1522.2 | 3739.2 | 452.6 | 2335.4 | 452.6 | 789.6 | 196.0 | – | 67.3 |
| HAB in CHS-Na MLV's | 6 | 5.0 | 500 | 128.5 | 151.1 | 2446.8 | 6755.9 | 1655.9 | 2281.7 | 722.0 | 1076.4 | 394.2 | 538.2 | – | 320.0 |
| HAB in CHS-TRIS MLV's | | 5.0 | 56 | 3.5 | 5.0 | 882.4 | 557.2 | 604.3 | 278.6 | 229.1 | 139.3 | – | – | – | – |
| HAB in CHS-TRIS MLV's | | 5.0 | 167 | 16.4 | 8.7 | 3064.9 | 1594.5 | 1324.1 | 350.6 | 557.7 | 452.6 | – | – | – | – |
| HAB in CHS-TRIS MLV's | 3 | 5.0 | 500 | 59.0 | 72.8 | 2383.1 | 2474.5 | 1038.6 | 1388.1 | 293.4 | 485.0 | 146.3 | 309.3 | – | 160.0 |
| HAB in CHS-TRIS MLV's | 5 | 5.0 | 1500 | 669.8 | 301.1 | 3442.7 | 5120.0 | 1991.5 | 1280.0 | 593.6 | 844.5 | – | – | – | – |
| Control | | 0 | NA | 0.1 | 5.0 | 0.1 | 5.0 | 0.2 | 5.0 | $<$0.3 | 5.0 | 0.1 | 5.0 | – | 5.0 |

Legend

Liposomes containing HA or HAB were prepared at the indicated lipid concentrations. (Example formulations are indicated) Entrapment values were determined by single radial immuno-diffusion (SRID) and liposomes were diluted in saline to a dosage of 0.5 or 5ug per 0.5 ml dose. Hartley guinea pigs were injected IM at 0 and 4 weeks. At the indicated timepoints, blood was collected by cardiac puncture. Serum antibody titers (total anti-HA IgG and neutralizing antibodies) were determined by EIA and HAI assay, respectively. Values represent the geometric mean of 3-5 guinea pigs per group.

EP 0 356 339 B1

Table 2

Effect of Liposome Entrapment of HAB on the Ability to Prime the Immune System

ANTI-HA ANTIBODY TITERS (Units/ml)

| Immunization | | Dose | Primary 4 week | | Secondary 6 week | | 8 week | | 12 week | | 23 week | | 52 week | |
| Primary | Boost | (ug) | EIA | HAI | EIA | HAI | EIA | HAI | EIA | HAI | EIA | HAI | EIA | HAI |
|---|---|---|---|---|---|---|---|---|---|---|---|---|---|---|
| **Experiment I** | | | | | | | | | | | | | | |
| HAB | HAB | 15 | 0.6 | 5.0 | 21.8 | 5.0 | 25.1 | 5.0 | 29.8 | 7.9 | 15.0 | 5.0 | 0.6 | 5.0 |
| Liposomal HAB | Liposomal HAB | 5 | 27.9 | 11.1 | 2706.4 | 1416.6 | 1287.1 | 1846.1 | 584.5 | 842.3 | 250.5 | 380.6 | 266.5 | 250.4 |
| Liposomal HAB | Liposomal HAB | 15 | 336.5 | 253.9 | 4112.7 | 2031.9 | 1060.8 | 2031.9 | 819.2 | 1280.0 | 312.5 | 320.0 | 258.8 | 226.3 |
| HAB | Liposomal HAB | 5 | 0.2 | 5.0 | 84.0 | 7.1 | 59.6 | 20.0 | 24.6 | 12.6 | 14.1 | 7.9 | 3.3 | 10.0 |
| Liposomal HAB | HAB | 5 | 7.7 | 6.6 | 711.1 | 595.6 | 397.5 | 932.1 | 169.7 | 595.6 | 154.5 | 269.1 | 97.6 | 269.1 |
| HA | HA | 5 | 11.6 | 16.8 | 719.4 | 466.1 | 266.0 | 500.8 | 166.8 | 134.5 | 24.9 | 40.0 | 18.5 | 31.8 |
| **Experiment II** | | | | | | | | | | | | | | |
| HAB | HAB | 5 | 0.4 | 5.0 | 1.8 | 5.0 | 1.4 | 5.0 | <1.5 | 9.1 | 1.4 | 6.3 | − | 5.0 |
| Liposomal HAB | Liposomal HAB | 5 | 59.0 | 72.8 | 2383.1 | 2474.5 | 1038.6 | 1388.1 | 293.4 | 485.0 | 146.3 | 309.3 | − | 160.0 |
| HA | HA | 5 | 24.9 | 28.6 | 527.3 | 1810.2 | 306.4 | 452.6 | 141.3 | 226.3 | 92.4 | 148.9 | − | 100.8 |
| Liposomal HAB | HAB | 5 | 26.9 | 10.0 | 1096.4 | 1208.4 | 561.5 | 320.0 | 326.2 | 250.4 | − | − | − | − |
| HA | HAB | 5 | 19.9 | 13.0 | 223.7 | 640.0 | 155.1 | 148.9 | 64.6 | 113.1 | − | − | − | − |

Legend

Liposomes containing HAB were prepared from CHS as in example 3. Entrapment value was determined by SRID and liposomes were diluted in saline to a dosage of 5 or 15 ug protein per 0.5 ml dose. Hartley guinea pigs were injected IM at 0 time and boosted at 4 weeks. At the indicated timepoints, blood was collected by cardiac puncture. Serum antibody titers (total anti-HA IgG and neutralizing antibodies) were determined by EIA and HAI assay, respectively. Values represent the geometric mean of 4-5 guinea pigs per group.

EP 0 356 339 B1

Table 3  Steroidal adjuvant effect after mixing or entrapping HAB

ANTI-HA antibody titers (Units/ml)

| Formulation | Primary 4 week | | Secondary 6 week | | 8 week | | 12 week | |
|---|---|---|---|---|---|---|---|---|
| | EIA | HAI | EIA | HAI | EIA | HAI | EIA | HAI |
| HA | 29.6 | 23.4 | 683.6 | 556.3 | 370.3 | 294.8 | 173.8 | 254.0 |
| HAB | 0.2 | 5.0 | 0.6 | 5.0 | 0.8 | 5.0 | 0.7 | 5.0 |
| HAB in CHS-Tris MLV's | 49.8 | 57.7 | 1998.3 | 842.3 | 1188.5 | 640.0 | – | – |
| HAB + CHS-Tris MLV's | 1.6 | 5.7 | 731.2 | 263.0 | 425.8 | 105.3 | – | – |
| HAB in CHS-Na MLV's | 34.1 | 37.8 | 2213.2 | 1280.0 | 799.6 | 604.2 | 39.7 | 485.0 |
| HAB + CHS-Na MLV's | 2.0 | 5.0 | 857.0 | 121.3 | 483.6 | 54.0 | – | – |

Legend:

HAB was entrapped in liposomes or mixed with empty liposomes.  All liposomes were prepared using 500 mg of lipid.
The inoculum contained 5 ug HAB or HA/0.5 ml.  The experiment was conducted as described in the legend of Table 1.

EP 0 356 339 B1

Table 4      Enhancement of Immunogenicity of HAB by use of DMPC:cholesterol SPLV's
at various mole ratios and lipid concentrations

| Formulation | DMPC: Cholesterol mole ratio | Starting Lipid concentration (mg) | Primary 4 week | | Secondary | | | |
|---|---|---|---|---|---|---|---|---|
| | | | EIA | HAI | 6 week EIA | 6 week HAI | 8 week EIA | 8 week HAI |
| HAB | NA | NA | 0.1 | 5.0 | 0.1 | 5.0 | 0.1 | 6.0 |
| HAB in Aluminum hydroxide gel | NA | NA | 0.1 | 5.0 | 1.8 | 5.0 | 8.0 | 5.0 |
| Liposomal HAB | 70:30 | 200 | 16.4 | 64.5 | 425.1 | 177.1 | 161.0 | 125.2 |
| | 50:50 | 50 | 7.1 | 4.0 | 171.0 | 5.0 | 119.3 | 5.0 |
| | 50:50 | 200 | 0.7 | 6.0 | 63.9 | 34.8 | 43.7 | 18.9 |
| | 50:50 | 500 | 31.1 | 23.4 | 200.1 | 47.6 | 89.0 | 40.0 |
| | 40:60 | 200 | 11.0 | 11.0 | 465.5 | 80.0 | 167.2 | 82.4 |
| | 30.70 | 200 | 18.7 | 5.0 | 492.2 | 56.6 | 259.9 | 36.6 |
| Control | NA | 0 | 0.1 | 5.5 | 0.1 | 5.0 | 0.1 | 5.0 |

Legend:

DMPC:Cholesterol SPLV's containing HAB were prepared at the indicated lipid concentrations and mole ratios. Entrapment values were determined by SRID and liposomes were diluted in saline to a dosage of 5ug per 0.5 ml dose. Hartley guinea pigs were injected i.m. at 0 and 4 weeks. At the indicated timepoints, blood was collected by cardiac puncture. Serum antibody titers (total anti-HA IgG and neutralizing antibodies) were determined by EIA and HAI assay, respectively. Values represent the geometric mean of 4-5 guinea pigs per group.

EP 0 356 339 B1

Table 5    Enhancement of Immunogenicity of HA by use of DMPC/cholesterol SPLV's

| Formulation | Dose (ug) | Primary 4 week | | Secondary | | | | | | | | | | |
|---|---|---|---|---|---|---|---|---|---|---|---|---|---|---|
| | | | | 6 week | | 8 week | | 12 week | | 25 week | | 52 week | | |
| | | EIA | RAI | EIA | RAI | EIA | RAI | EIA | RAI | EIA | RAI | EIA | RAI |
| HA | 0.5 | 0.9 | 5.0 | 103.4 | 190.3 | 43.5 | 31.3 | 29.6 | 40.0 | 29.5 | 40.0 | - | 23.7 |
| Liposomal HA | 0.5 | 72.6 | 26.4 | 1938.3 | 3469.8 | 1561.7 | 905.1 | 28.2 | 354.1 | 429.3 | 269.1 | - | 105.3 |
| HA | 5.0 | 24.9 | 20.6 | 527.3 | 1810.2 | 306.4 | 452.6 | 141.3 | 226.3 | 92.4 | 148.9 | - | 100.8 |
| Liposomal HA | 5.0 | 349.1 | 398.6 | 5480.1 | 8914.4 | 3649.7 | 3189.1 | 2233.9 | 1940.1 | 1121.8 | 864.6 | - | 320.0 |

Legend:

DMPC/cholesterol SPLV's (70:30 mole ratio) containing HA were prepared as in Example 1.  Entrapment values were determined by SRID and Liposomes were diluted in saline to a dosage of 0.5 or 5.0 ug per 0.5 ml dose.  Hartley guinea pigs were injected i.m. at 0 and 4 weeks.  At the indicated time points, blood was collected by cardiac puncture.  Serum antibody titers (total anti-HA IgG and neutralizing antibodies) were determined by EIA and RAI assay, respectively.  Values represent the geometric mean of 4-5 guinea pigs per group.

Table 6 Enhancement of Immunogenicity of HAB by use of DMPC:Cholesterol SPLV's and Aluminum Hydroxide gel.

| Formulation | Dose (ug) | Starting Lipid Concentration | Primary 4 week EIA | HAI | Secondary 6 week EIA | HAI | 8 week EIA | HAI | 12 week EIA | HAI | 25 week EIA | HAI | 52 week EIA | HAI |
|---|---|---|---|---|---|---|---|---|---|---|---|---|---|---|
| **Experiment I** | | | | | | | | | | | | | | |
| HAB | 5.0 | NA | 0.3 | 5.0 | 5.9 | 5.7 | 2.5 | 5.0 | | | | | | |
| Liposomal HAB | 5.0 | 200 | 15.6 | 7.6 | 596.5 | 60.6 | 132.8 | 30.3 | | | | | | |
| Liposomal HAB in Aluminum hydroxide gel | 5.0 | 200 | 18.5 | 9.4 | 1093.7 | 80.0 | 123.4 | 30.3 | | | | | | |
| Liposomal HAB | 5.0 | 500 | 54.6 | 32.8 | 2738.4 | 183.8 | 460.1 | 100.0 | | | | | | |
| Liposomal HAB in Aluminum hydroxide gel | 5.0 | 500 | 48.7 | 15.2 | 7546.1 | 297.8 | 557.5 | 100.8 | | | | | | |
| **EXPERIMENT II** | | | | | | | | | | | | | | |
| HAB (in saline) | 5.0 | NA | 0.4 | 5.0 | 1.8 | 5.0 | 1.4 | 5.0 | <1.5 | 9.1 | 1.4 | 6.3 | - | 5.0 |
| Liposomal HAB** | 5.0 | 500 | 18.3 | 11.5 | 363.7 | 183.8 | 163.8 | 47.6 | - | - | - | - | - | - |
| Liposomal HAB in Aluminum hydroxide gel** | 5.0 | 500 | 73.3 | 34.8 | 1045.8 | 970.1 | 435.4 | 422.2 | 172.9 | 242.5 | 178.3 | 302.1 | - | 40.0 |
| Liposomal HAB in Aluminum hydroxide gel** | 0.5 | 500 | 1.8 | 5.0 | 335.9 | 163.8 | 102.7 | 60.6 | 22.7 | 63.5 | - | - | - | - |

Legend:

DMPC:Cholesterol SPLV's (70:30 mole ratio) containing HAB were prepared at the indicated lipid concentrations. The liposomes in Experiment II were sterile filtered through a 0.4/0.2 um filter stack. Entrapment values were determined by SRID and liposomes were diluted in saline or aluminum hydroxide gel and saline to a dosage of 0.5 or 5 ug per 0.5 ml dose. Hartley guinea pigs were injected i.m. at 0 and 4 weeks. At the indicated timepoints, blood was collected by cardiac puncture. Serum antibody titers (total anti-HA IgG and neutralizing antibodies) were determined by EIA and HAI assay, respectively. Values represent the geometric mean of 4-5 guinea pigs per group.

EP 0 356 339 B1

Table 7

ANTI-HA ANTIBODIES IN SERA OF MICE IMMUNIZED WITH HA ENTRAPPED IN LIPOSOMES

| HA FORMULATION | DOSE/0.5 ml/INOCULUM PROTEIN(ug), LIPID(mg) | | 4 week | | 6 week | | 8 week | | 12 week | |
|---|---|---|---|---|---|---|---|---|---|---|
| | | | EIA | HAI | EIA | HAI | EIA | HAI | EIA | HAI |
| Non-Entrapped | 5.0 | – | 26 | 29 | 516 | 557 | 301 | 368 | 198 | 320 |
| | 0.5 | – | 3 | 9 | 205 | 243 | 126 | 211 | 57 | 106 |
| MLV, CHST, H | 5.0 | 13.6 | 891 | 761 | 7062 | 7241 | 2170 | 3044 | 1581 | 1318 |
| | 0.5 | 1.4 | 52 | 33 | 2655 | 2941 | 1018 | 1237 | 557 | 640 |
| MLV, CHST, L | 5.0 | 1.9 | 147 | 92 | 3886 | 5553 | 3055 | 1810 | 1166 | 1191 |
| | – | – | – | – | – | – | – | – | – | – |
| SPLV, DMPC/C, H | 5.0 | 9.7 | 123 | 80 | 2204 | 1940 | 1548 | 1470 | 776 | 640 |
| | 0.5 | 1.0 | 22 | 23 | 387 | 399 | 233 | 243 | 136 | 121 |
| SPLV, DMPC/C, L | 5.0 | 2.9 | 101 | 89 | 1766 | 1810 | 1042 | 1613 | 767 | 905 |
| | – | – | – | – | – | – | – | – | – | – |

A/ Liposomes (MLV, SPLV) were prepared with either 500 mg (H) or 50 mg (L) of lipid (CHST or DMPC/C, 70:30 mole percent)

B/ Geometric mean titer (n-51) determined by enzyme-linked immuno assay (EIA) and hemagglutination imhibition (HAI)

EP 0 356 339 B1

**Claims**

**Claims for the following Contracting States : AT, BE, CH, DE, FR, GB, IT, LI, LU, NL, SE**

1. A dosage form comprising an influenza antigen and a multilamellar liposome comprising dimyristoylphosphatidylcholine (DMPC) and cholesterol, wherein said antigen and liposome are present in an immunization dose.

2. A dosage form comprising an antigen and a multilamellar liposome comprising dimyristoylphosphatidyl-choline (DMPC) and cholesterol, wherein the molar ratio of DMPC to cholesterol is from 65:35 to 80:20 and wherein said antigen and liposome are present in an immunization dose.

3. The dosage form of Claim 1 or 2 further comprising aluminum adjuvant.

4. The dosage form of Claim 3 wherein the aluminum adjuvant is aluminum hydroxide gel.

5. The dosage form of Claim 1 wherein the DMPC and cholesterol are present in a DMPC/cholesterol molar ratio of between 80:20 and 20:80.

6. The dosage form of Claim 5 wherein said molar ratio is between 70:30 and 30:70.

7. The dosage form of any one of Claims 1 to 6 wherein said molar ratio is 70:30 ± 5.

8. The dosage form of any one of Claims 1 to 7 wherein said liposome is of equal solute distribution.

9. The dosage form of Claim 8 wherein said liposome is an SPLV.

10. The dosage form of any one of Claims 1 to 9 wherein said liposome is at least 1 micron in diameter.

11. The dosage form of Claim 1 wherein said antigen is selected from the group comprising proteins, peptides, polysaccharides, nucleic acids, lipids, glycolipids, lipoproteins, lipopolysaccharides, synthetic peptides or bacterial, viral, protozoal, tissue, or cellular fractions.

12. The dosage form of Claim 11 wherein said antigen is an influenza virus fraction or a fragment thereof.

13. The dosage form of Claim 12 wherein said influenza virus fraction is the hemagglutinin fraction or a fragment thereof.

14. The dosage form of Claim 13 wherein said antigen is the bromelain fragment of the hemagglutinin fraction of an influenza virus.

15. The dosage form of any one of Claims 1 to 14 further comprising an immunomodulator.

16. The dosage form of Claim 15 wherein the immunomodulator is a cytokine.

17. The dosage form of any one of Claims 1 to 16 further comprising a suitable pharmaceutical carrier.

18. A composition for potentiating or priming an immune response in an animal, including a human, comprising an immunization dose of a dosage form of Claims 1 to 17 and a pharmaceutical vehicle.

**Claims for the following Contracting States : ES, GR**

1. A process for preparing a dosage form comprising associating an influenza antigen and a multilamellar liposome comprising dimyristoylphosphatidylcholine (DMPC) and cholesterol, wherein said antigen and liposome are present in an immunization dose.

2. A process for preparing a dosage form comprising an antigen and a multilamellar liposome comprising dimyristoylphosphatidylcholine (DMPC) and cholesterol, wherein the molar ratio of DMPC to cholesterol is from 65:35 to 80:20 and wherein said antigen and liposome are present in an immunization dose.

EP 0 356 339 B1

**3.** The process for preparing the dosage form of Claim 1 or 2 further comprising aluminum adjuvant.

**4.** The process for preparing the dosage form of Claim 3 wherein the aluminum adjuvant is aluminum hydroxide gel.

**5.** The process for preparing the dosage form of Claim 1 wherein the DMPC and cholesterol are present in a DMPC/Cholesterol molar ratio of between 80:20 and 20:80.

**6.** The process for preparing the dosage form of Claim 5 wherein said molar ratio is between 70:30 and 30:70.

**7.** The process for preparing the dosage form of any one of Claims 1 to 6 wherein said molar ratio is 70:30 ± 5.

**8.** The process for preparing the dosage form of any one of Claims 1 to 7 wherein said liposome is of equal solute distribution.

**9.** The process for preparing the dosage form of Claim 8 wherein said liposome is an SPLV.

**10.** The process for preparing the dosage form of any one of Claims 1 to 9 wherein said liposome is at least 1 Micron in diameter.

**11.** The process for preparing the dosage form of Claim 1 wherein said antigen is selected from the group comprising proteins, peptides, polysaccharides, nucleic acids, lipids, glycolipids, lipoproteins, lipopolysaccharides, synthetic peptides or bacterial, viral, protozoal, tissue, or cellular fractions.

**12.** The process for preparing the dosage form of Claim 11 wherein said antigen is an influenza virus fraction or a fragment thereof.

**13.** The process for preparing the dosage form of claim 12 wherein said influenza virus fraction is the hemagglutinin fraction or a fragment thereof.

**14.** The process for preparing the dosage form of Claim 13 wherein said antigen is the bromelain fragment of the hemagglutinin fraction of an influenza virus.

**15.** The process for preparing the dosage form of any one of Claims 1 to 14 further comprising an immunomodulator.

**16.** The process for preparing the dosage form of Claim 15 wherein the immunomodulator is a cytokine.

**17.** The process for preparing the dosage form of any one of Claims 1 to 16 further comprising a suitable pharmaceutical carrier.

**18.** A process for preparing a composition for potentiating or priming an immune response in an animal, including a human, comprising associating an immunization dose of a dosage form of Claims 1 to 17 and a pharmaceutical vehicle.

**Patentansprüche**
**Patentansprüche für folgende Vertragsstaaten : AT, BE, CH, DE, FR, GB, IT, LI, LU, NL, SE**

**1.** Dosierungsform, die umfaßt ein Influenza-Antigen und ein multilamellares Liposom, enthaltend Dimyristoylphosphatidylcholin (DMPC) und Cholesterin, worin das genannte Antigen und das genannte Liposom in einer Immunisierungsdosis vorliegen.

**2.** Dosierungsform, die umfaßt ein Antigen und ein multilamellares Liposom, enthaltend Dimyristoylphosphatidylcholin (DMPC) und Cholesterin, worin das Molverhältnis von DMPC zu Cholesterin 65:35 bis 80:20 beträgt und das genannte Antigen und das genannte Liposom in einer Immunisierungsdosis vorliegen.

22

3. Dosierungsform nach Anspruch 1 oder 2, die außerdem ein Aluminium-Adjuvans umfaßt.

4. Dosierungsform nach Anspruch 3, worin das Aluminium-Adjuvans Aluminiumhydroxid-Gel ist.

5. Dosierungsform nach Anspruch 1, worin das DMPC und das Cholesterin in einem DMPC/Cholesterin-Molverhältnis zwischen 80:20 und 20:80 vorliegen.

6. Dosierungsform nach Anspruch 5, worin das genannte Molverhältnis zwischen 70:30 und 30:70 liegt.

7. Dosierungsform nach einem der Ansprüchen 1 bis 6, worin das genannte Molverhältnis 70:30 ± 5 beträgt.

8. Dosierungsform nach einem der Ansprüchen 1 bis 7, worin das genannte Liposom eine gleichmäßige Verteilung des gelösten Stoffes aufweist.

9. Dosierungsform nach Anspruch 8, worin das genannte Liposom ein SPLV ist.

10. Dosierungsform nach einem der Ansprüchen 1 bis 9, worin das genannte Liposom einen Durchmesser von mindestens 1 $\mu$m hat.

11. Dosierungsform nach Anspruch 1, worin das genannte Antigen ausgewählt wird aus der Gruppe, die umfaßt Proteine, Peptide, Polysaccharide, Nucleinsäuren, Lipide, Glycolipide, Lipoproteine, Lipopolysaccharide, synthetische Peptide oder bakterielles, virales oder protozoales Gewebe oder Zellfraktionen.

12. Dosierungsform nach Anspruch 11, worin das genannte Antigen eine Influenzavirus-Fraktion oder ein Fragment davon ist.

13. Dosierungsform nach Anspruch 12, worin die genannte Influenzavirus-Fraktion die Hämagglutinin-Fraktion oder ein Fragment davon ist.

14. Dosierungsform nach Anspruch 13, worin das genannte Antigen das Bromelain-Fragment der Hämagglutinin-Fraktion eines Influenzavirus ist.

15. Dosierungsform nach einem der Ansprüche 1 bis 14, die außerdem einen Immunmodulator umfaßt.

16. Dosierungsform nach Anspruch 15, worin der Immunmodulator ein Cytokin ist.

17. Dosierungsform nach einem der Ansprüche 1 bis 16, die außerdem einen geeigneten pharmazeutischen Träger umfaßt.

18. Zusammensetzung zur Potenzierung oder Auslösung einer Immunantwort in einem Tier einschließlich eines Menschen, die umfaßt eine Immunisierungsdosis einer Dosierungsform nach den Ansprüchen 1 bis 17 und ein pharmazeutisches Vehiculum.

**Patentansprüche für folgende Vertragsstaaten : ES, GR**

1. Verfahren zur Herstellung einer Dosierungsform, das umfaßt das Assoziieren (Kombinieren) eines Influenza-Antigens mit einem multilamellaren Liposom, enthaltend Dimyristoylphosphatidylcholin (DMPC) und Cholesterin, wobei das genannte Antigen und das genannte Liposom in einer Immunisierungsdosis vorliegen.

2. Verfahren zur Herstellung einer Dosierungsform, die umfaßt ein Antigen und ein multilamellares Liposom, enthaltend Dimyristoylphosphatidylcholin (DMPC) und Cholesterin, worin das genannte Antigen und das genannte Liposom in einer Immunisierungsdosis vorliegen.

3. Verfahren zur Herstellung der Dosierungsform nach Anspruch 1 oder 2, die außerdem ein Aluminium-Adjuvans umfaßt.

4. Verfahren zur Herstellung der Dosierungsform nach Anspruch 3, worin das Aluminium-Adjuvans Aluminiumhydroxid-Gel ist.

5. Verfahren zur Herstellung der Dosierungsform nach Anspruch 1, worin das DMPC und das Cholesterin in einem DMPC/Cholesterin-Molverhältnis zwischen 80:20 und 20:80 vorliegen.

6. Verfahren zur Herstellung der Dosierungsform nach Anspruch 5, worin das genannte Molverhältnis zwischen 70:30 und 30:70 liegt.

7. Verfahren zur Herstellung der Dosierungsform nach einem der Ansprüchen 1 bis 6, worin das genannte Molverhältnis 70:30 ± 5 beträgt.

8. Verfahren zur Herstellung der Dosierungsform nach einem der Ansprüchen 1 bis 7, worin das genannte Liposom eine gleichmäßige Verteilung des gelösten Stoffes aufweist.

9. Verfahren zur Herstellung der Dosierungsform nach Anspruch 8, worin das genannte Liposom ein SPLV ist.

10. Verfahren zur Herstellung der Dosierungsform nach einem der Ansprüchen 1 bis 9, worin das genannte Liposom einen Durchmesser von mindestens 1 µm hat.

11. Verfahren zur Herstellung der Dosierungsform nach Anspruch 1, worin das genannte Antigen ausgewählt wird aus der Gruppe, die umfaßt Proteine, Peptide, Polysaccharide, Nucleinsäuren, Lipide, Glycolipide, Lipoproteine, Lipopolysaccharide, synthetische Peptide oder bakterielles, virales oder protozoales Gewebe oder Zellfraktionen.

12. Verfahren zur Herstellung der Dosierungsform nach Anspruch 11, worin das genannte Antigen eine Influenzavirus-Fraktion oder ein Fragment davon ist.

13. Verfahren zur Herstellung der Dosierungsform nach Anspruch 12, worin die genannte Influenzavirus-Fraktion die Hämagglutinin-Fraktion oder ein Fragment davon ist.

14. Verfahren zur Herstellung der Dosierungsform nach Anspruch 13, worin das genannte Antigen das Bromelain-Fragment der Hämagglutinin-Fraktion eines Influenzavirus ist.

15. Verfahren zur Herstellung der Dosierungsform nach einem der Ansprüche 1 bis 14, die außerdem einen Immunmodulator umfaßt.

16. Verfahren zur Herstellung der Dosierungsform nach Anspruch 15, worin der Immunmodulator ein Cytokin ist.

17. Verfahren zur Herstellung der Dosierungsform nach einem der Ansprüche 1 bis 16, die außerdem einen geeigneten pharmazeutischen Träger umfaßt.

18. Verfahren zur Herstellung einer Zusammensetzung zur Potenzierung oder Auslösung einer Immunantwort in einem Tier einschließlich eines Menschen, das umfaßt das Assoziieren (Kombinieren) einer Immunisierungsdosis einer Dosierungsform nach den Ansprüchen 1 bis 17 mit einem pharmazeutischen Vehiculum.

**Revendications**
**Revendications pour les Etats contractants suivants : AT, BE, CH, DE, FR, GB, IT, LI, LU, NL, SE**

1. Une forme de dosage comprenant un antigène de la grippe et un liposome multilamellaire comportant de la dimyristoylphosphatidylcholine (DMPC) et du cholestérol, dans laquelle ledit antigène et ledit liposome sont présents dans une dose d'immunisation.

2. Une forme de dosage comportant un antigène et un liposome multilamellaire renfermant de la dimyristoylphosphatidylcholine (DMPC) et du cholestérol, dans laquelle le rapport molaire de la DMPC au cholestérol est de 65:35 à 80:20 et dans laquelle ledit antigène et le liposome sont présents dans une dose d'immunisation.

3. La forme de dosage de la revendication 1 ou 2, comportant en outre un adjuvant à base d'aluminium.

4. La forme de dosage de la revendication 3, dans laquelle l'adjuvant à base d'aluminium est un gel d'hydroxyde d'aluminium.

5. La forme de dosage de la revendication 1, dans laquelle la DMPC et le cholestérol sont présents selon un rapport molaire DMPC/cholestérol se situant entre 80:20 et 20:80.

6. La forme de dosage de la revendication 5, dans laquelle ledit rapport molaire se situe entre 70:30 et 30:70.

7. La forme de dosage de l'une quelconque des revendications 1 à 6, dans laquelle ledit rapport molaire est de 70:30 ± 5.

8. La forme de dosage de l'une quelconque des revendications 1 à 7, dans laquelle ledit liposome présente une distribution égale en soluté.

9. La forme de dosage de la revendication 8, dans laquelle ledit liposome est une SPLV (vésicule stable plurilamellaire).

10. La forme de dosage de l'une quelconque des revendications 1 à 9, dans laquelle ledit liposome présente un diamètre d'au moins 1 micron.

11. La forme de dosage de la revendication 1, dans laquelle ledit antigène est choisi dans le groupe comportant des protéines, des peptides, des polysaccharides, des acides nucléiques, des lipides, des glycolipides, des lipoprotéines, des lipopolysaccharides, des peptides synthétiques ou des fractions bactériennes, virales, protozoaires, de tissu ou cellulaires.

12. La forme de dosage de la revendication 11, dans laquelle ledit antigène est une fraction de virus grippal ou un fragment de celui-ci.

13. La forme de dosage de la revendication 12, dans laquelle ladite fraction de virus grippal est la fraction d'hémagglutinine ou un fragment de celle-ci.

14. La forme de dosage de la revendication 13, dans laquelle ledit antigène est le fragment bromelaine de la fraction hémagglutinine d'un virus grippal.

15. La forme de dosage de l'une quelconque des revendications 1 à 14, comportant en outre un immunomodulateur.

16. La forme de dosage de la revendication 15, dans laquelle l'immunomodulateur est une cytokine.

17. La forme de dosage de l'une quelconque des revendications 1 à 16, comportant en outre un support pharmaceutique approprié.

18. Une composition pour potentialiser ou favoriser une réponse immunitaire chez un animal, y compris un être humain, comportant une dose d'immunisation d'une forme de dosage selon les revendications 1 à 17 et un véhicule pharmaceutique.

**Revendications pour les Etats contractants suivants : ES, GR**

1. Un procédé pour préparer une forme de dosage comprenant un antigène de la grippe et un liposome multilamellaire comportant de la dimyristoylphosphatidylcholine (DMPC) et du cholestérol, dans lequel ledit antigène et ledit liposome sont présents dans une dose d'immunisation.

2. Un procédé pour préparer une forme de dosage comportant un antigène et un liposome multilamellaire renfermant de la dimyristoylphosphatidylcholine (DMPC) et du cholestérol, dans lequel ledit antigène et le liposome sont présents dans une dose d'immunisation.

3. Le procédé pour préparer la forme de dosage de la revendication 1 ou 2, comportant en outre un adjuvant à base d'aluminium.

4. Le procédé pour préparer la forme de dosage de la revendication 3, dans lequel l'adjuvant à base d'aluminium est un gel d'hydroxyde d'aluminium.

5. Le procédé pour préparer la forme de dosage de la revendication 1, dans lequel la DMPC et le cholestérol sont présents selon un rapport molaire DMPC/cholestérol se situant entre 80:20 et 20:80.

6. Le procédé pour préparer la forme de dosage de la revendication 5, dans lequel ledit rapport molaire se situe entre 70:30 et 30:70.

7. Le procédé pour préparer la forme de dosage de l'une quelconque des revendications 1 à 6, dans lequel ledit rapport molaire est de 70:30 ± 5.

8. Le procédé pour préparer la forme de dosage de l'une quelconque des revendications 1 à 7, dans lequel ledit liposome présente une distribution égale en soluté.

9. Le procédé pour préparer la forme de dosage de la revendication 8, dans lequel ledit liposome est une SPLV (vésicule stable plurilamellaire).

10. Le procédé pour préparer la forme de dosage de l'une quelconque des revendications 1 à 9, dans lequel ledit liposome présente un diamètre d'au moins 1 micron.

11. Le procédé pour préparer la forme de dosage de la revendication 1, dans lequel ledit antigène est choisi dans le groupe comportant des protéines, des peptides, des polysaccharides, des acides nucléiques, des lipides, des glycolipides, des lipoprotéines, des lipopolysaccharides, des peptides synthétiques ou des fractions bactériennes, virales, protozoaires, de tissu ou cellulaires.

12. Le procédé pour préparer la forme de dosage de la revendication 11, dans lequel ledit antigène est une fraction de virus grippal ou un fragment de celui-ci.

13. Le procédé pour préparer la forme de dosage de la revendication 12, dans lequel ladite fraction de virus grippal est la fraction d'hémagglutinine ou un fragment de celle-ci.

14. Le procédé pour préparer la forme de dosage de la revendication 13, dans lequel ledit antigène est le fragment bromelaine de la fraction hémagglutinine d'un virus grippal.

15. Le procédé pour préparer la forme de dosage de l'une quelconque des revendications 1 à 14, comportant en outre un immunomodulateur.

16. Le procédé pour préparer la forme de dosage de la revendication 15, dans lequel l'immunomodulateur est une cytokine.

17. Le procédé pour préparer la forme de dosage de l'une quelconque des revendications 1 à 16, comportant en outre un support pharmaceutique approprié.

18. Un procédé pour préparer une composition pour potentialiser ou favoriser une réponse immunitaire chez un animal, y compris un être humain, procédé selon lequel on associe une dose d'immunisation d'une forme de dosage selon les revendications 1 à 17 et un véhicule pharmaceutique.